**Europäisches Patentamt**

**European Patent Office**

**Office européen des brevets**

(19)

(11) Numéro de publication: **0 275 771**
**B1**

(12) # FASCICULE DE BREVET EUROPEEN

(45) Date de publication du fascicule du brevet:
09.08.89

(21) Numéro de dépôt: **87402923.4**

(22) Date de dépôt: **18.12.87**

(51) Int. Cl.⁴: **C07C 69/63**, C07C 67/11, C07C 31/40, C07C 29/124

(54) **Procédé de préparation des tétrahydro-1,1,2,2, perfluoroalcanols et de leurs esters.**

(30) Priorité: **22.12.86 FR 8617983**

(43) Date de publication de la demande:
**27.07.88 Bulletin 88/30**

(45) Mention de la délivrance du brevet:
**09.08.89 Bulletin 89/32**

(84) Etats contractants désignés:
**AT BE CH DE ES FR GB IT LI NL SE**

(56) Documents cités:
**EP-A- 0 024 224**
**DE-A- 3 035 641**
**US-A- 3 239 557**

(73) Titulaire: **ATOCHEM, 4 & 8, Cours Michelet La Défense 10, F-92800 Puteaux(FR)**

(72) Inventeur: **Lantz, André, Domaine de la Hétraie 552, route de Charly, F-69390 Vernaison(FR)**
Inventeur: **Michaud, Pascal, 5 Chemin de la Bastéro Les Chênes, F-69350 La Mulatière(FR)**

(74) Mandataire: **Leboulenger, Jean et al, ATOCHEM Département Propriété Industrielle, F-92091 Paris la Défense 10 Cédex 42(FR)**

**Description**

La présente invention concerne la synthèse des tétrahydro-1,1,2,2 perfluoroalcanols ($R_FCH_2CH_2$-OH) et de leurs esters par oxydation des iodures de perfluoroalkyl-2 éthyle correspondants ($R_FCH_2$-$CH_2I$) au moyen du peroxyde d'hydrogène. Le radical perfluoroalkyle $R_F$ peut contenir de 1 à 20 atomes de carbone et être à chaîne linéaire ou ramifiée.

Ces alcools et esters polyfluorés sont des intermédiaires intéressants pour la fabrication d'agents tensioactifs et de produits hydrophobes et oléophobes. Ils peuvent en particulier être facilement transformés en esters acryliques ou méthacryliques dont la polymérisation, éventuellement avec d'autres monomères, fournit des agents d'apprêt hydrophobe et oléophobe pour les matières textiles, le cuir, le papier ou d'autres substrats. Les mélanges d'alcools et d'esters peuvent aussi être transformés entièrement en alcools.

Plusieurs procédés de préparation de ces alcools et esters polyfluorés sont déjà connus. Le procédé décrit dans le brevet FR 1 380 579 qui consiste à faire réagir un iodure $R_FCH_2CH_2I$ avec de l'oléum puis à hydrolyser le sulfate formé, présente l'inconvénient de fournir des quantités importantes de diesters sulfuriques qui sont difficilement hydrolysables.

Selon un procédé décrit dans le brevet US 3 239 557 il est possible d'obtenir les esters $R_FCH_2CH_2OCOR$ par réaction d'un iodure $R_FCH_2CH_2I$ avec un sel d'acide carboxylique RCOOH. Les rendements ne sont cependant pas très bons car il se forme des quantités plus ou moins importantes d'oléfine $R_FCH=CH_2$.

Les alcools $R_FCH_2CH_2OH$ peuvent aussi être obtenus selon le procédé du brevet FR 2 096 179 qui consiste à préparer les nitrates $R_FCH_2CH_2ONO_2$ par réaction des iodures $R_FCH_2CH_2I$ avec l'acide nitrique et à hydrogéner ces nitrates en alcool. Ce procédé présente toutefois l'inconvénient de nécessiter deux étapes réactionnelles dont la dernière doit être réalisée sous une pression élevée d'hydrogène.

Le brevet FR 2 180 113 décrit un procédé de fabrication de mélanges d'alcools $R_FCH_2CH_2OH$ et de formiates $R_FCH_2CH_2OCOH$ par réaction à haute température des iodures $R_FCH_2CH_2I$ avec le diméthylformamide en présence d'un peu d'eau. Ce procédé présente l'inconvénient d'exiger des conditions de réaction très sévères et de fournir comme sous-produit l'oléfine $R_FCH=CH_2$, ce qui diminue d'autant le rendement. Une bonne sélectivité en alcool et formiate ne peut en outre être obtenue qu'en utilisant des quantités très importantes de diméthylformamide.

Plus récemment, ou a proposé dans les brevets EP 0024224 et DE 3 035 641 de préparer ces alcools et esters polyfluorés en faisant réagir l'iodure $R_FCH_2CH_2I$ avec un peracide $RCO_3H$ préalablement formé par l'addition de peroxyde d'hydrogène à un acide carboxylique $RCO_2H$ en présence ou non d'une faible quantité d'acide sulfurique. Cependant, dans les conditions opératoires décrites, ce procédé conduit à une sous-production d'oléfine $R_FCH=CH_2$ en quantité non négligeable et/ou à un faible taux de conversion de l'iodure $R_FCH_2CH_2I$.

Il a maintenant été trouvé un procédé permettant de pallier ces inconvénients, c'est-à-dire d'éviter une sous-production d'oléfine $R_FCH=CH_2$ et d'obtenir néanmoins un taux de conversion très élevé.

Le procédé selon l'invention qui consiste à oxyder un iodure de perfluoroalkyl-2 éthyle au moyen du peroxyde d'hydrogène au sein d'un acide carboxylique ou d'un ester d'un tel acide et en présence d'acide sulfurique est caractérisé en ce que, pour une mole d'iodure de perfluoroalkyl-2 éthyle, on met en oeuvre de 1 à 30 moles d'acide sulfurique (de préférence, de 3 à 10 moles), de 1 à 50 moles d'acide carboxylique ou d'ester d'un tel acide (de préférence, de 5 à 15 moles), et de 3 à 20 moles de peroxyde d'hydrogène (de préférence, 5 à 6 moles).

Le peroxyde d'hydrogène est avantageusement utilisé sous forme de solutions aqueuses dont la concentration en $H_2O_2$ peut varier d'environ 35 à 75 % en poids et est de préférence comprise entre 65 et 75 %.

Bien qu'on préfère utiliser l'acide sulfurique pur ou très concentré (80 % en poids et plus), on peut également employer des solutions d'acide sulfurique contenant jusqu'à 50 % en poids d'eau.

Comme acides carboxyliques, on utilise de préférence les acides aliphatiques liquides dans les conditions opératoires ; ces acides qui contiennent généralement de 1 à 8 atomes de carbone peuvent être linéaires ou ramifiés, saturés ou non, et contenir des substituants tels que, par exemple, des atomes d'halogène. Conviennent particulièrement bien l'acide acétique et l'acide propionique.

On peut également utiliser des acides carboxyliques solides tels que les acides aliphatiques lourds ou les acides aromatiques (par exemple l'acide benzoïque et ses dérivés substitués) en ajoutant un solvant tel qu'un alcool (par exemple, méthanol, éthanol, propanol) ou un ester.

Comme indiqué précédemment, on peut utiliser un ester d'acide carboxylique. Cet ester est de préférence un ester d'alcool aliphatique contenant 1 à 4 atomes de carbone, par exemple l'acétate d'éthyle, l'acétate de butyle ou le propionate d'éthyle.

L'oxydation selon l'invention peut être effectuée à une température pouvant aller de -20 à 140°C, mais avantageusement comprise entre 60 et 90°C.

Le procédé selon l'invention peut être réalisé de différentes façons. On peut par exemple opérer comme dans les brevets EP 0024224 et DE 3 035 641 en ajoutant au iodure de perfluoroalkyl-2 éthyle un mélange de peroxyde d'hydrogène, d'acide sulfurique et d'acide carboxylique ou d'ester dans les proportions requises. Il est également possible d'opérer de façon inverse en introduisant l'iodure dans un tel mélange.

Cependant, le mode de réalisation préféré de l'invention consiste à introduire le peroxyde d'hydrogène dans un mélange de l'iodure de perfluoroalkyl-2 éthyle, d'acide sulfurique et de l'acide ou ester carboxylique. Cette introduction est avantageusement effectuée sous agitation énergique avec un débit tel que la température du milieu réactionnel se maintienne d'elle-même entre 60 et 90°C.

La réaction est en général très rapide et se fait avec une libération d'iode et/ou d'acide iodique qu'on peut séparer facilement du mélange réactionnel par simple filtration. La majeure partie de l'iode peut ainsi être récupérée sous forme d'iode élémentaire par traitement de l'acide iodique avec un réducteur classique tel que le sulfite de sodium.

Les produits fluorés peuvent êtres isolés suivant les méthodes habituelles, par exemple par décantation et lavage de la phase organique à l'eau. On obtient finalement un produit constitué majoritairement par l'ester de perfluoroalkyl-2 éthyle ($R_FC_2H_4OCOR$) qu'on peut saponifier pour obtenir l'alcool $R_FCH_2CH_2OH$ ou transformer en un autre ester, notamment en acrylate ou méthacrylate.

## AVERTISSEMENT

Comme cela est déjà signalé dans le brevet DE 3.035.641 précité, le travail avec les peracides et le peroxyde d'hydrogène comporte d'importants risques d'inflammation et d'explosion. Il conviendra donc, lors de la mise en oeuvre du procédé selon l'invention, de prendre à cet égard toutes les mesures de précaution usuelles.

Les exemples suivants dans lesquels les pourcentages s'entendent en poids, illustrent l'invention sans la limiter.

## EXEMPLE 1

Dans un réacteur en verre de 250 ml, muni d'un agitateur énergique, d'un réfrigérant à reflux et d'une ampoule de coulée, on charge 47,4 g (0,1 mole) d'iodure de perfluorohexyl-2 éthyle, 60 g d'acide acétique et 28 ml d'acide sulfurique à 98 %, puis on ajoute goutte à goutte en 50 minutes 24,7 g d'une solution à 70 % de peroxyde d'hydrogène (soit 0,5 mole de $H_2O_2$). La température s'élève d'elle-même à 75-80°C et est maintenue à cette valeur pendant encore 30 à 45 minutes après la fin de l'addition.

Le mélange réactionnel est ensuite filtré pour séparer l'iode et l'acide iodique formés, puis on décante le filtrat et on lave la phase organique trois fois avec 50 ml d'eau à 25°C.

On obtient ainsi 38 g de phase organique dont la répartition des composés fluorés, déterminée par chromatographie, est la suivante :
95 % de $C_6F_{13}C_2H_4OCOCH_3$
3,1 % de $C_6F_{13}C_2H_4OH$
1,5 % de $(C_6F_{13}C_2H_4O)_2SO_2$
0,4 % de $C_6F_{13}C_2H_4I$ non transformé.

## EXEMPLE 2

Dans un réacteur identique à celui de l'exemple 1, on charge 47,4 g d'iodure de perfluorohexyl-2 éthyle, 30 ml d'acide sulfurique à 98 % et 74 g d'acide propionique, puis on ajoute goutte à goutte en 45 minutes 26,9 g d'une solution à 70 % de peroxyde d'hydrogène et on maintient ensuite pendant encore 30 minutes à 75-80°C sous agitation.

Après filtration, décantation du filtrat et lavage au sulfite de sodium et à l'eau, on récupère 40 g de phase organique dont la répartition des composés fluorés est la suivante :
98,4 % de $C_6F_{13}C_2H_4OCOC_2H_5$
1,4 % de $C_6F_{13}C_2H_4OH$
0,2 % de $C_6F_{13}C_2H_4I$ non transformé.

## EXEMPLE 3

En opérant comme à l'exemple 2 à partir de 37,4 g d'iodure de perfluorobutyl-2 éthyle, on obtient 28 g de phase organique dont la répartition des composés fluorés est la suivante :
97 % de $C_4F_9C_2H_4OCOC_2H_5$
1,5 % de $C_4F_9C_2H_4OH$
1,5 % de $C_4F_9C_2H_4I$ non transformé.

## EXEMPLE 4

On opère comme à l'exemple 2, mais en remplaçant l'iodure de perfluorohexyl-2 éthyle par 53,8 g d'un mélange d'iodures $R_FC_2H_4I$ ayant la composition pondérale suivante :

| $R_F$ | % |
| --- | --- |
| $C_6F_{13}$ | 56,1 |
| $C_8F_{17}$ | 25,0 |
| $C_{10}F_{21}$ | 10,1 |
| $C_{12}F_{25}$ | 4,0 |
| $\geq C_{14}F_{29}$ | 4,8 |

le poids moléculaire moyen de ce mélange étant voisin de 538.

La répartition des composés fluorés dans la phase organique ainsi obtenue (40 g) est la suivante :
97,8 % de $R_FC_2H_4OCOC_2H_5$
2 % de $R_FC_2H_4OH$
0,2 % de $R_FC_2H_4I$ non transformés.

## EXEMPLE 6

En opérant comme à l'exemple 2 à partir de 27,4 g de iodure de pentafluoro-3,3,4,4,4 butyle, on obtient 20 g de phase organique dont les composés fluorés se répartissent comme suit :
96,5 % de $C_2F_5C_2H_4OCOC_2H_5$
1,5 % de $C_2F_5C_2H_4OH$
2 % de $C_2F_5C_2H_4I$ non transformé.

## EXEMPLE 6

Dans un réacteur identique à celui de l'exemple 1, on charge 47,4 g d'iodure de perfluorohexyl-2 éthyle, 30 ml d'acide sulfurique à 98 % et 163 g d'acide trichloroacétique. On ajoute ensuite sous agitation 26,9 g d'une solution à 70 % de peroxyde d'hydrogène à un débit tel que la température se maintienne à 70-80°C. Quand l'addition du peroxyde d'hydrogène est terminée, on laisse sous agitation pendant encore 30 minutes.

Aprés refroidissement à 20°C, on ajoute 100 ml d'eau. Par filtration, on récupère 12,3 g d'iode brut. Aprés décantation du filtrat et lavage de la phase organique à l'eau, on obtient 53 g d'un produit dont la répartition des composés fluorés est la suivante :
90,6 % de $C_6F_{13}C_2H_4OCOCCl_3$
1,1 % de $C_6F_{13}C_2H_4OH$
5,6 % de $(C_6F_{13}C_2H_4O)_2SO_2$
2,7 % de $C_6F_{13}C_2H_4I$ non transformé.

### EXEMPLE 7

On opère comme à l'exemple 2, main en remplaçant les 74 g d'acide propionique par 144 g d'acide octanoïque. On obtient ainsi 56 g de phase organique dont les composés fluorés se répartissent comme suit :
98 % de $C_6F_{13}C_2H_4OCOC_7H_{15}$
0,8 % de $C_6F_{13}C_2H_4OH$
1,2 % de $C_6F_{13}C_2H_4I$ non transformé.

### EXEMPLE 8

On répète l'exemple 2, main en remplaçant l'acide propionique par 88 g d'acétate d'éthyle. On obtient alors 38 g de phase organique dont les composés fluorés se répartissent comme suit :
79,3 % de $C_6F_{13}C_2H_4OCOCH_3$
18,2 % de $C_6F_{13}C_2H_4OH$
2,5 % de $C_6F_{13}C_2H_4I$ non transformé.

### Revendications

1. Procédé de préparation des tétrahydro-1,1,2,2 perfluoroalcanols et de leurs esters par oxydation d'un iodure de perfluoroalkyl-2 éthyle au moyen du peroxyde d'hydrogène au sein d'un acide carboxylique ou d'un ester d'un tel acide et en présence d'acide sulfurique, caractérisé en ce que, pour une mole d'iodure de perfluoroalkyl-2 éthyle, on met en oeuvre de 1 à 30 moles d'acide sulfurique, de 1 à 50 moles d'acide carboxylique ou d'ester d'un tel acide, et de 3 à 20 moles de peroxyde d'hydrogène.

2. Procédé selon la revendication 1, dans lequel on met en oeuvre 3 à 10 moles d'acide sulfurique, 5 à 15 moles d'acide carboxylique ou d'ester d'un tel acide, et 5 à 6 moles de peroxyde d'hydrogène par mole d'iodure de perfluoroalkyl-2 éthyle.

3. Procédé selon la revendication 1 ou 2, dans lequel la réaction est effectuée à une température de 60 à 90°C.

4. Procédé selon l'une des revendications 1 à 3, dans lequel on introduit le peroxyde d'hydrogène dans une solution comprenant le iodure de perfluoroalkyle-2 éthyle, l'acide carboxylique ou un ester d'un tel acide, et l'acide sulfurique.

5. Procédé selon l'une des revendications 1 à 4, dans lequel le peroxyde d'hydrogène est utilisé sous forme d'une solution aqueuse dont la concentration en $H_2O_2$ peut varier de 35 à 75 % en poids, de préférence entre 65 et 75 %.

6. Procédé selon l'une des revendications 1 à 5, dans lequel on utilise l'acide sulfurique pur ou dilué avec jusqu'à 50 % en poids d'eau.

7. Procédé selon l'une des revendications 1 à 6, dans lequel on met en oeuvre l'acide acétique ou propionique.

### Patentansprüche

1. Verfahren zur Herstellung der 1,1,2,2-Tetrahydroperfluoralkanole und ihrer Ester durch Oxidation eines 2-Perfluoralkylethyljodids mit Hilfe von Wasserstoffperoxid in Lösung einer Carbonsäure oder eines Esters einer solchen Säure und in Gegenwart von Schwefelsäure, dadurch gekennzeichnet, daß man pro Mol 2-Perfluoralkylethyljodid 1 bis 30 Mol Schwefelsäure, 1 bis 50 Mol Carbonsäure oder Carbonsäureester und 3 bis 20 Mol Wasserstoffperoxid umsetzt.

2. Verfahren nach Anspruch 1, bei dem man 3 bis 10 Mol Schwefelsäure, 5 bis 15 Mol Carbonsäure oder Carbonsäureester und 5 bis 6 Mol Wasserstoffperoxid pro Mol 2-Perfluoralkylethyljodid umsetzt.

3. Verfahren nach Anspruch 1 oder 2, bei dem die Reaktion bei einer Temperatur von 60 bis 90°C durchgeführt wird.

4. Verfahren nach einem der Ansprüche 1 bis 3, bei dem man das Wasserstoffperoxid zu einer Lösung enthaltend das 2-Perfluoralkylethyljodid, die Carbonsäure oder den Carbonsäureester und die Schwefelsäure zugibt.

5. Verfahren nach einem der Ansprüche 1 bis 4, bei dem das Wasserstoffperoxid in Form einer wässrigen Lösung verwendet wird, deren Konzentration an $H_2O_2$ zwischen 35 und 75 Gew.-%, vorzugsweise zwischen 65 und 75 Gew.-% variieren kann.

6. Verfahren nach einem der Ansprüche 1 bis 5, bei dem man die Schwefelsäure rein oder mit bis zu 50 Gew.-% Wasser verdünnt verwendet.

7. Verfahren nach einem der Ansprüche 1 bis 6, bei dem man Essigsäure oder Propionsäure umsetzt.

### Claims

1. Process for the preparation of 1,1,2,2-tetrahydroperfluoroalkanols and of their esters by oxidation of a 2-(perfluoroalkyl)ethyl iodide by means of hydrogen peroxide in a carboxylic acid or an ester of such an acid and in the presence of sulphuric acid, characterized in that 1 to 30 moles of sulphuric acid, 1 to 50 moles of carboxylic acid or of an ester of such an acid and 3 to 20 moles of hydrogen peroxide are employed per one mole of 2-(perfluoroalkyl)ethyl iodide.

2. Process according to claim 1, in which 3 to 10 moles of sulphuric acid, 5 to 15 moles of carboxylic acid or of an ester of such an acid, and 5 to 6 moles of hydrogen peroxide are employed per mole of 2-(perfluoroalkyl)ethyl iodide.

3. Process according to claim 1 or 2, in which the reaction is carried out at a temperature of 60 to 90°C.

4. Process according to one of claims 1 to 3, in which the hydrogen peroxide is introduced into a solution comprising the 2-(perfluoroalkyl)ethyl iodide, the carboxylic acid or an ester of such an acid, and the sulphuric acid.

5. Process according to one of claims 1 to 4, in which the hydrogen peroxide is employed in the form of an aqueous solution whose $H_2O_2$ concentration can vary from 35 to 75% by weight, preferably between 65 and 75%.

6. Process according to one of claims 1 to 5, in which the sulphuric acid is employed pure or diluted with up to 50% by weight of water.

7. Process according to one of claims 1 to 6, in which acetic or propionic acid is employed.